# EUROPEAN PATENT APPLICATION

(11) **EP 1 632 922 A1**
(43) Date of publication of application: **08.03.2006**
(21) Application number: 04734114.4
(22) Date of filing: 20.05.2004
(51) Int. Cl.: G08C 17/02, A61B 5/00

(54) **DATA COLLECTION SYSTEM AND DATA COLLECTION METHOD**

(30) Priority: 21.05.2003 JP 2003143787; 27.02.2004 JP 2004054657
(71) Applicant: JMS Co., Ltd., Hiroshima-shi, Hiroshima 730-8652 (JP); Nakamoto, Hidetomo, Tokyo 179-0085 (JP)
(72) Inventor: NAKAMOTO, Hidetomo, Nerima-ku, Tokyo 179-0085 (JP); YOSHIMOTO, Mitsuo, c/o JMS Co., Ltd., Hiroshima-shi, Hiroshima 730-8652 (JP); HATTA, Masato, c/o JMS Co., Ltd., Hiroshima-shi, Hiroshima 730-8652 (JP); RYUZAKI, Munekazu, Meguro-ku, Tokyo 152-0032 (JP); SONE, Masayoshi, Shibuya-ku, Tokyo 150-0001 (JP); NISHIDA, Eiichi, Kitakyushu-shi, Fukuoka 805-0016 (JP)
(74) Representative: Grape, Knut
(86) International application number: PCT/JP2004/007218
(87) International publication number: WO 2004/104965

(57) **Abstract**

A data collection system includes: a server (7) connected to the Internet (6); a plurality of data acquisition target devices (1); and a plurality of data transfer devices (3) that can be connected to the data acquisition target devices, respectively. Device data acquired by each data transfer device from each data acquisition target device are transferred to the server via the Internet using a mobile phone (5). The data transfer device includes: a data acquisition part (13) for acquiring the device data from the data acquisition target device; a data transfer part (14) for combining transmission instruction data with the device data so as to generate transfer data, and transferring the transfer data to the mobile phone; and a control part (15) for allowing the operation of acquiring the device data and the operation of transferring the transfer data to be executed. The transmission instruction data correspond to an instruction to the mobile phone to perform an operation of transmitting the device data to a predetermined server.

## Description

### Technical Field

The present invention relates to a data collection system and a data collection method for collecting device data obtained by, for example, a sphygmomanometer, via the Internet.

### Background Art

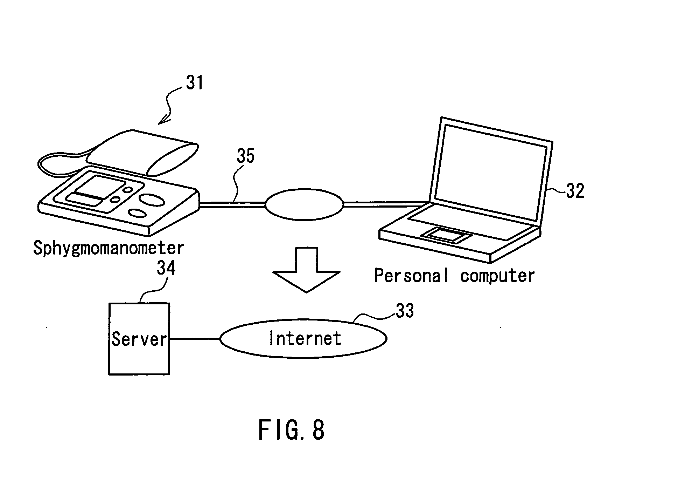

Figure 8 is a view schematically showing a configuration of a conventional device data collection system. This data collection system includes a sphygmomanometer 31 for measuring patient's blood pressure, a personal computer 32, and a server 34 connected to the Internet 33 for accumulating blood pressure data.

The sphygmomanometer 31 is connected to the personal computer 32 via a serial cable 35. The personal computer 32 can be connected to the server 34 via the Internet 33. With this data collection system, blood pressure data indicating patient's blood pressure measured by the sphygmomanometer 31 can be accumulated in the server 34.

In the data collection system thus configured, when the patient's blood pressure is measured by the sphygmomanometer 31, the blood pressure data indicating the measured blood pressure are transferred to the personal computer 32 through the serial cable 35. The blood pressure data transferred to the personal computer 32 are accumulated in the server 34 via the Internet 33.

The above-mentioned data collection system facilitates the collection of the blood pressure data of patients in remote areas, and enables doctors to diagnose the health condition related to the blood pressure of the patients in remote areas (see, for example, JP 2002-355305 A).

However, according to the data collection system with the above configuration, in order to transfer the patient's blood pressure data measured by the sphygmomanometer 31 to the server 34, the operation of the personal computer 32 is necessary for establishing a connection to the Internet 33. The personal computer 32 has a long starting time before it becomes operational since the power has been turned on. Further, in order to operate software for transferring the blood pressure data to the server 34, complicated operations of input devices such as a mouse, a keyboard, and the like are involved.

Patients to be diagnosed using the data collection system as described above are often elderly people, who are unfamiliar with the operation of the personal computer and the like, and thus complicated operations have a serious impact on their use of the system. Consequently, patients in remote areas have difficulty in transferring their own blood pressure data to the server 34 via the Internet 33.

### Disclosure of Invention

It is an object of the present invention to provide a data collection system that allows device data such as blood pressure data obtained by a device such as a sphygmomanometer provided in a remote area to be transferred easily to a server connected to the Internet.

A data collection system according to the present invention includes: a server connected to the Internet; a plurality of data acquisition target devices; and a plurality of data transfer devices that are capable of being connected to the data acquisition target devices, respectively, wherein device data acquired by each data transfer device from each data acquisition target device are transferred to the server via the Internet using a mobile phone. The data transfer device includes: a data acquisition part for acquiring the device data from the data acquisition target device; a data transfer part for combining transmission instruction data with the device data so as to generate transfer data, and transferring the transfer data to the mobile phone; and a control part for allowing the data acquisition part to execute the operation of acquiring the device data and allowing the data transfer part to execute the operation of transferring the transfer data. The transmission instruction data correspond to an instruction to the mobile phone to perform an operation of transmitting the device data to a predetermined server via the Internet.

A data collection method for collecting data according to the present invention uses a server connected to the Internet, a plurality of data acquisition target devices, a plurality of data transfer devices that are capable of being connected to the data acquisition target devices, respectively, and a mobile phone that is capable of being connected to the Internet, the method including transferring device data acquired from each data acquisition target device from the mobile phone to the server via the Internet. The method uses the same data transfer devices as used in the data collection system according to the present invention.

### Brief Description of Drawings

Figure 1 is a block diagram schematically showing a configuration of a data collection system according to an embodiment of the present invention.
Figure 2 is a block diagram showing a configuration of a data transfer device for use in the configuration of the system.
Figure 3 is a perspective exterior view of the data transfer device.
Figure 4 is a view showing a screen on which blood pressure data and the like accumulated in a server of the data collection system according to the present embodiment are displayed in graphical form.
Figure 5 is a view showing a screen on which the blood pressure data and the like accumulated in the server of the data collection system are displayed numerically.
Figure 6 is a view showing a screen on which data of a patient using a sphygmomanometer of the data collection system are displayed.
Figure 7 is a view showing a screen on which a list of patients using the sphygmomanometer of the data collection system is displayed.
Figure 8 is a view schematically showing a configuration of a conventional data collection system.

### Description of the Invention

According to a data collection system according to the present invention, device data obtained by a device such as a sphygmomanometer provided in a remote area can be collected easily from a mobile phone into a predetermined server via the Internet.

It is possible that the data transfer device includes a starting switch, and the control part starts the operations of the data acquisition part and the data transfer part when the starting switch is operated.

Preferably, the data transfer device further includes a memory for storing a serial number for identifying the data transfer device mutually, and the data transfer part adds the serial number to the device data so as to generate data to be transmitted to the server.

It is possible that the data transfer part includes an emulator for converting data into an emulation code in accordance with a keyboard emulation format for operating the mobile phone, so as to convert the transfer data into the emulation code and to transfer the converted data.

In this configuration, it is possible that the data transfer part includes a data generation part and a transfer processing part, the data generation part generates data including the device data and the transmission instruction data and supplies the generated data to the transfer processing part, and the transfer processing part converts the data supplied from the data generation part into the emulation code and outputs the converted data from a data transfer terminal.

In the above configuration, the control part may have a function of detecting whether or not a good connection state is established between the data acquisition target device and the mobile phone, and a display part may be provided so that whether or not a good connection state is established is shown by a blinking state controlled by the control part.

The data acquisition target device can be a medical instrument, an electric meter for measuring a consumption of electricity, a water meter for measuring a consumption of tap water, a production facility that is capable of outputting log data, or a household electric appliance that is capable of outputting data indicating an operating state. The medical instrument can be a sphygmomanometer, a pulsimeter, or a glucometer.

It is possible that the data acquisition target device is a medical instrument, medicine-taking information indicating whether or not the patient takes a predetermined medicine is capable of being transmitted to the server using the mobile phone, and the server includes a medicine-taking condition judgment part for accumulating the medicine-taking information and transmitting a warning mail concerning the taking of the medicine to the mobile phone based on the medicine-taking information.

It is possible that the medicine-taking condition judgment part transmits the warning mail when the medicine is taken at a rate less than a predetermined rate, or when the medicine is not taken for a predetermined number of consecutive days.

In the above configuration, it is possible that a doctor's computer connected to the Internet further is provided for analyzing the data accumulated in the server, the data acquisition target device includes a sphygmomanometer for measuring patient's blood pressure, and the data include blood pressure data measured by the sphygmomanometer, the server includes an abnormal blood pressure value treatment part for transmitting an abnormality report mail to the doctor's computer when an abnormality is found in the blood pressure data, the doctor's computer transmits a treatment instruction to the abnormal blood pressure value treatment part based on the abnormality report mail, and the abnormal blood pressure value treatment part transmits a treatment instruction mail to the mobile phone based on the treatment instruction.

In this configuration, it is possible that the abnormal blood pressure value treatment part transmits one of a plurality of kinds of treatment instruction mails prepared in advance to the mobile phone. Further, the treatment instruction mail can include a description of instructing the patient to change a dosage of a medicine to take.

In the above configuration, it is possible that a doctor's computer connected to the Internet further is provided for analyzing the data accumulated in the server, the data acquisition target device includes a sphygmomanometer for measuring patient's blood pressure, and the data include blood pressure data measured by the sphygmomanometer, harmful symptom information indicating patient's harmful symptom is capable of being transmitted from the mobile phone to the server, the server includes a harmful symptom treatment part for transmitting a harmful symptom report to the doctor's computer based on the harmful symptom information, the doctor's computer transmits a treatment instruction to the harmful symptom treatment part based on the harmful symptom report, and the harmful symptom treatment part transmits a treatment instruction mail to the mobile phone based on the treatment instruction.

Hereinafter, an embodiment of the present invention will be described specifically with reference to the drawings.

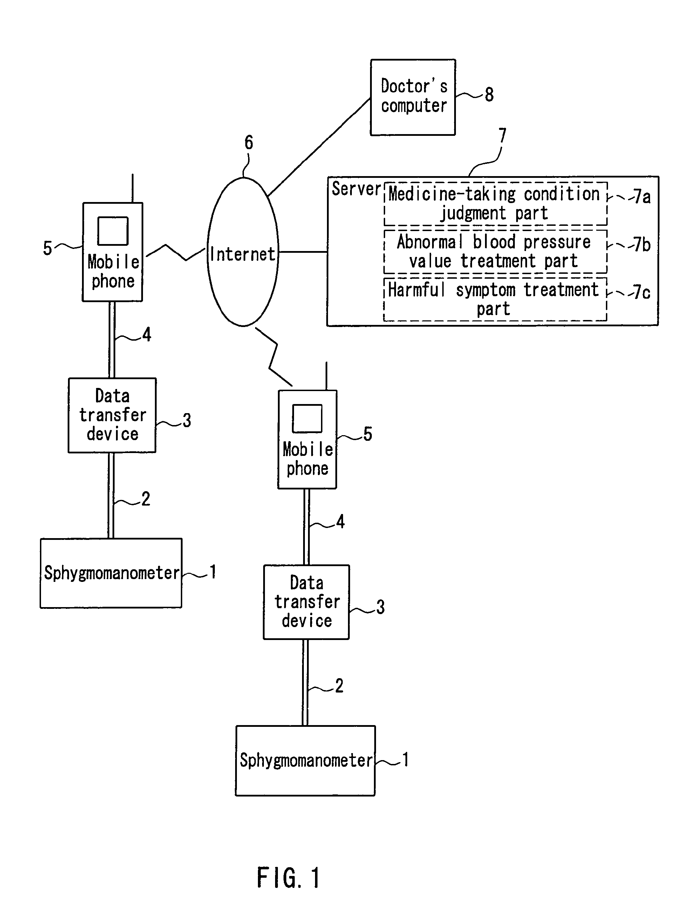

Figure 1 schematically shows a configuration of a data collection system according to the present embodiment. This data collection system collects blood pressure data indicating patient's measured blood pressure from a plurality of sphygmomanometers 1. Each of the sphygmomanometers 1 is connected with a data transfer device 3 via a serial cable 2. The data transfer device 3 is connected to a mobile phone 5 via a transfer cable 4. The mobile phone 5 can be connected to the Internet 6.

The data collection system further includes a server 7 connected to the Internet 6 for accumulating the blood pressure data measured by each of the sphygmomanometers 1, and a doctor's computer 8 that can be connected to the Internet 6. The doctor's computer 8 is provided to allow a doctor to analyze the blood pressure data accumulated in the server 7.

The server 7 includes a medicine-taking condition judgment part 7a, an abnormal blood pressure value treatment part 7b, and a harmful symptom treatment part 7c. The medicine-taking condition judgment part 7a transmits a warning mail concerning the taking of a medicine to the mobile phone 5 based on medicine-taking information indicating whether or not a patient using the sphygmomanometer 1 takes a predetermined medicine. To this end, the patient transmits the medicine-taking information to the server 7 using the mobile phone 5, and the server 7 accumulates the medicine-taking information. When an abnormality is found in the patient's blood pressure data measured by the sphygmomanometer 1 and transferred via the mobile phone 5, the abnormal blood pressure value treatment part 7b transmits an abnormality report mail to the doctor's computer 8. The harmful symptom treatment part 7c transmits a harmful symptom report to the doctor's computer 8 based on harmful symptom information indicating the patient's harmful symptom transmitted from the mobile phone 5.

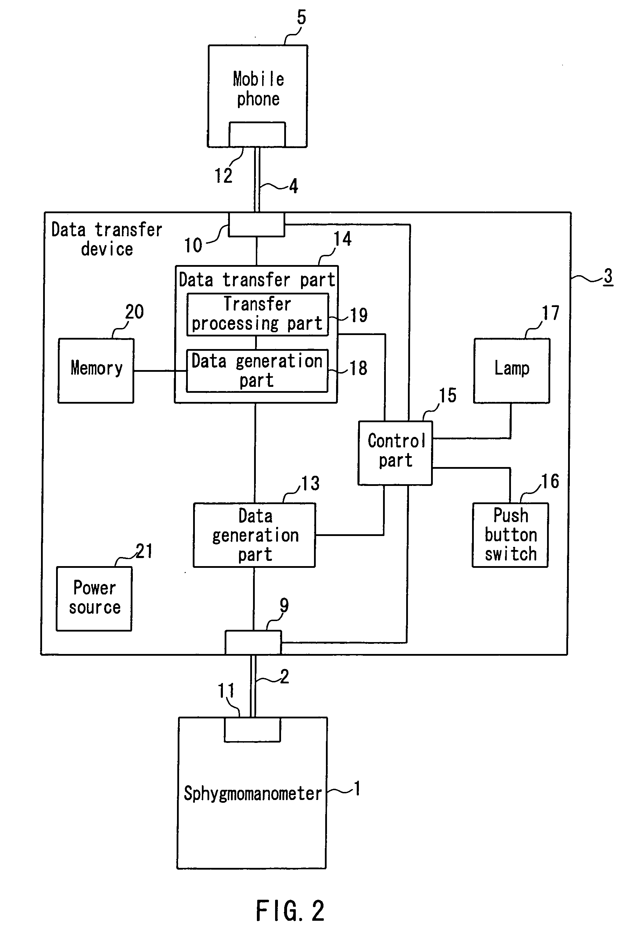

Figure 2 is a block diagram showing a configuration of the data transfer device 3 for use in the data collection system of the present embodiment, and a connection structure between the data transfer device 3 and the sphygmomanometer 1 as well as the mobile phone 5. The data transfer device 3 includes a serial terminal 9 to be connected with one end of the serial cable 2, and a transfer terminal 10 to be connected with one end of the transfer cable 4. The other end of the serial cable 2 is connected with a serial terminal 11 provided in the sphygmomanometer 1. The other end of the transfer cable 4 is connected with a terminal 12 of the mobile phone 5. Each of the cables can be connected/disconnected freely with/from the terminal.

The data transfer device 3 includes as main components a data acquisition part 13, a data transfer part 14, and a control part 15. These components can be constituted by a microprocessor. The data acquisition part 13 is supplied with the output of the sphygmomanometer 1 via the serial terminal 11, the serial cable 2, and the serial terminal 9. The output of the data acquisition part 13 is supplied to the data transfer part 14. The output of the data transfer part 14 is supplied to the mobile phone 5 via the transfer terminal 10, the transfer cable 4, and the transfer terminal 12. The control part 15 controls the operations of the data acquisition part 13 and the data transfer part 14.

The control part 15, which is connected with a push button switch 16, performs a control so that a series of operations of the data acquisition part 13 and the data transfer part 14 is started, when the push button switch 16 is operated. The control part 15 is connected with the serial terminal 9 and the transfer terminal 10 to detect whether or not the serial cable 2 and the transfer cable 4 are in a good connection state. The detection may be performed by any usual methods, and thus a specific description thereof will be omitted. The result of the detection is indicated by a lamp 17.

Under the control of the control part 15, the data acquisition part 13 is started and acquires sphygmomanometer data including the measured blood pressure data and data for specifying the date and time at which the blood pressure was measured from the sphygmomanometer 1 and supplies the acquired data to the data transfer part 14.

The data transfer part 14 includes a data generation part 18 and a transfer processing part 19. The data transfer part 14 is connected with a memory 20, in which a serial number for identifying the data transfer device 3 and transmission instruction data are stored. The data generation part 18 adds the serial number and the transmission instruction data obtained from the memory 20 to the sphygmomanometer data obtained from the data acquisition part 13 so as to generate transfer data, and supplies the transfer data to the transfer processing part 19. The transfer processing part 19 converts the transfer data into an emulation code representing a state where a key or the like of the mobile phone 5 is being pushed, in accordance with a keyboard emulation format, and transfers the converted data to the mobile phone 5.

In the mobile phone 5, processing of establishing a connection to the Internet 6 and processing of transmitting the sphygmomanometer data and the serial number to the server 7 are performed based on the transmission instruction data included in the transferred data. As a result, the sphygmomanometer data and the serial number are transferred from the mobile phone 5 to the server 7 via the Internet 6.

The processing of the transfer from the mobile phone 5 is performed based on, for example, the POST/GET method of the Internet. To this end, the transmission instruction data stored in the memory 20 of the data transfer device 3 include data concerning a menu selection of the mobile phone 5, the activation of a function of the mobile phone 5 for establishing a connection to the Internet as well as the connection, and the input of a URL of the server as a transfer destination. The transfer processing part 19 converts the transmission instruction data including the above data, numerical values of the sphygmomanometer data, and the serial number into an emulation code, and outputs the converted data. When the mobile phone 5 has an emulator, the conversion processing by the transfer processing part 19 is not necessary.

A power source 21 provided in the data transfer device 3 is constituted by, for example, a dry battery, and supplies a voltage to operate the data acquisition part 13, the data transfer part 14, the control part 15, the lamp 17, and the memory 20.

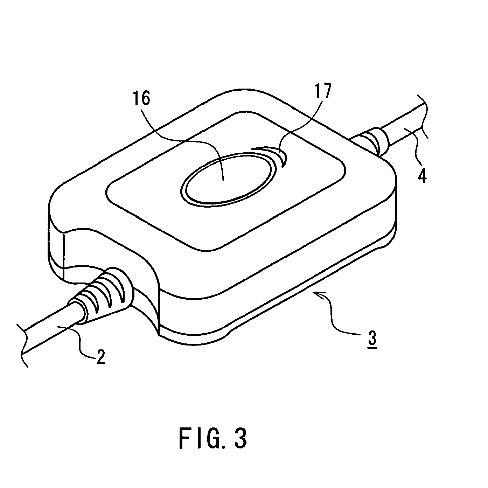

Figure 3 is a perspective exterior view of the data transfer device 3 according to the present embodiment. The data transfer device 3 is substantially rectangular, and has the push button switch 16 with an elliptical shape in the center of a top surface thereof. The lamp 17 with a crescent shape is provided next to the push button switch 16.

The operation of the data collection system thus configured will be described below. When the power source of the data transfer device 3 is turned on, the control part 15 detects whether or not the serial cable 2 and the transfer cable 4 are in a good connection state. When a good connection state is established, the lamp 17 is lighted. When either one of the serial cable 2 and the transfer cable 4 is in a bad connection state, the lamp 17 is blinking. With this configuration, the connection state of the serial cable 2 or the transfer cable 4 can be modified, so that an error in transferring the sphygmomanometer data due to a poor connection can be prevented.

Next, when the patient pushes down the push button switch 16, the data acquisition part 13 acquires the sphygmomanometer data measured by the sphygmomanometer 1 by means of serial communications. The acquired sphygmomanometer data are supplied to the data transfer part 14.

Then, in the data transfer part 14, initially the data generation part 18 adds the serial number and the transmission instruction data stored in the memory 20 to the sphygmomanometer data, and supplies the obtained data to the transfer processing part 19. The transfer processing part 19 converts the supplied data in accordance with a keyboard emulation format of the mobile phone 5, and transfers the converted data to the mobile phone 5.

In accordance with the transmission instruction data included in the transfer data received from the data transfer part 14, the mobile phone 5 performs the operation for establishing a connection to the Internet 6 and the operation of transmitting the sphygmomanometer data and the serial number to the server 7 via the Internet 6. Then, the server 7 receives and accumulates the sphygmomanometer data and the serial number transmitted from the mobile phone 5.

As a result, it becomes possible that the doctor's computer 8 acquires the sphygmomanometer data accumulated in the server 7 via the Internet 6 and analyzes the same.

As described above, according to the data collection system of the present embodiment, the data transfer device 3 transfers the sphygmomanometer data measured by the sphygmomanometer 1 from the mobile phone 5 to the server 7 via the Internet 6 almost automatically. Therefore, it is possible to transmit the sphygmomanometer data simply, without using a personal computer involving complicated operations. More specifically, the operation of the data transfer device 3 is only to establish connections using the serial cable 2 and the transfer cable 4 and to push the push button switch 16 once, which is extremely simple.

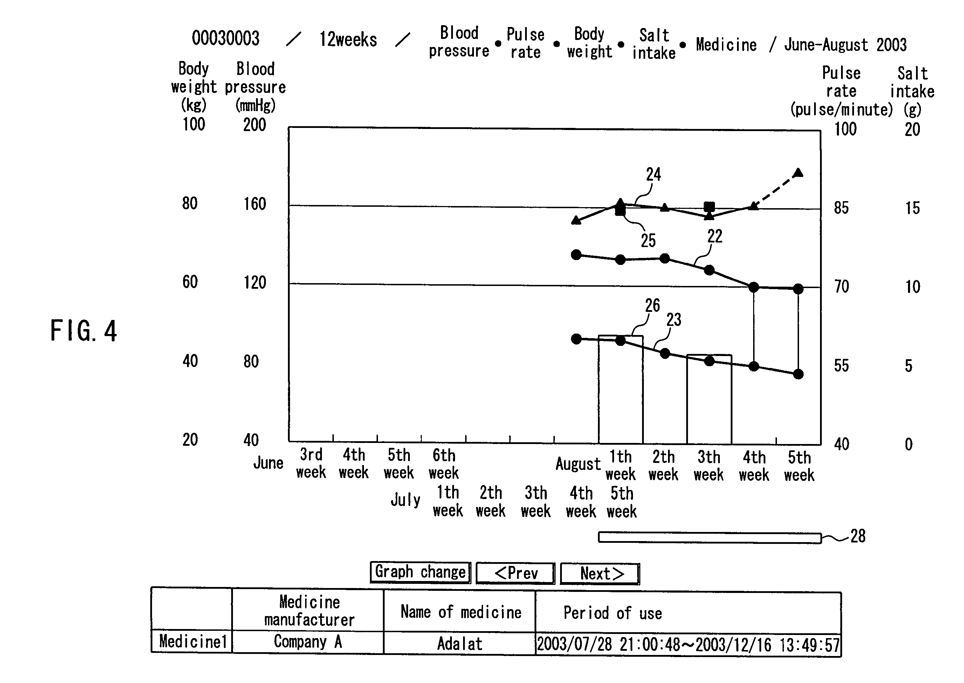

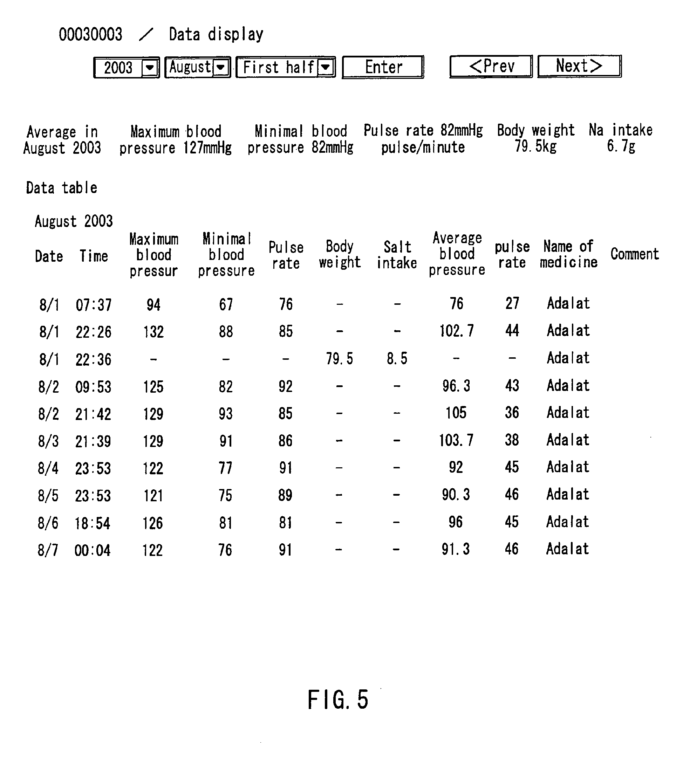

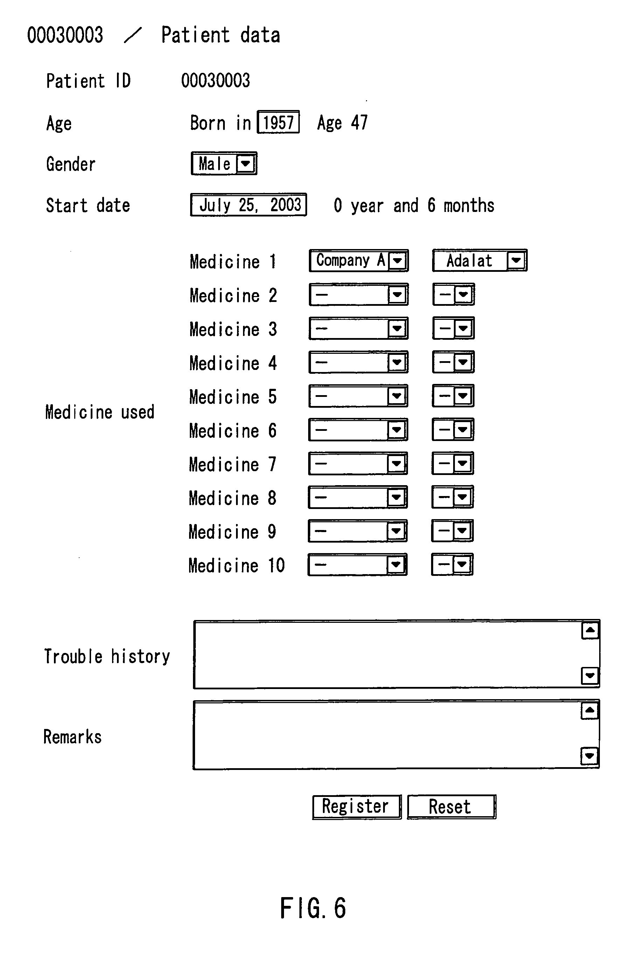

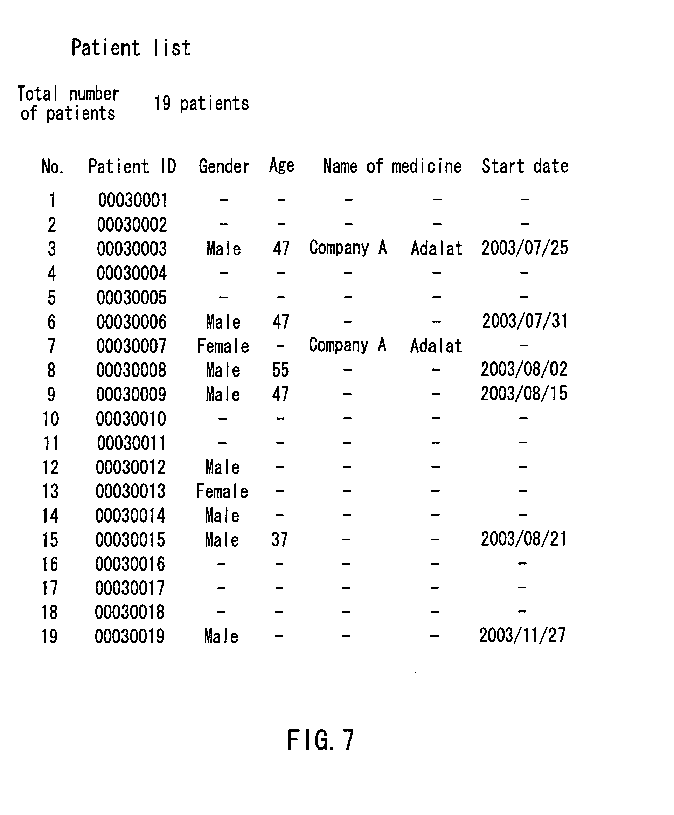

Next, a function of the doctor's computer 8 will be described. The doctor's computer 8 acquires the blood pressure data accumulated in the server 7 via the Internet 6 and analyzes the same. Figure 4 shows a screen on which the blood pressure data accumulated in the server 7 are displayed by the doctor's computer 8 in graphical form. Figure 5 shows a screen on which the data of a blood pressure, body weight, a pulse rate, and the like are displayed numerically, or a name of a medicine taken and the like is displayed. Figure 6 shows a screen on which data of a history, a medical history, a history of taking the medicine, and the like of the patient using the sphygmomanometer 1 are displayed. Figure 7 shows a screen on which a list of patients using the data collection system is displayed.

As shown in Figures 4 and 5, the patient is identified by a patient ID, and age, gender, the start date of taking the medicine, the name of a manufacturer of the medicine, and the name of the medicine are displayed on the screen.

Figures 4 and 5 show the data of the patient whose patient ID is 00030003 between 07:37 on August 1 and 00:04 on August 7, 2003. In Figure 4, • on a kinked line 22 represent data of the maximum blood pressure, and • on a kinked line 23 represent data of the minimal blood pressure. On a kinked line 24, ▲ represent weight data, and ■ represent pulse rate data of the patient. Further, vertical bars 26 represent salt intake data. These data are constituted so that they can be input to the mobile phone 5 if necessary and transferred to the server 7. On the screen in Figure 5, the data on the screen in Figure 4 are displayed numerically.

Further, it is displayed on the screen in Figure 4 that the patient takes one kind of medicine (medicine 1), whose manufacturer is "company A" and whose name is "Adalat". Further, it is shown that the medicine is used during a period between 21:00:48 on July 28, 2003 and 13:49:57 on December 16, 2003. A horizontal bar 28 in Figure 4 represents the period during which the medicine 1 is taken.

The medicine-taking information indicating whether or not the patient takes a medicine to be taken can be input from the mobile phone 5. The input medicine-taking information is transmitted from the mobile phone 5 to the server 7. The medicine-taking condition judgment part 7a provided in the server 7 transmits a warning mail concerning the taking of the medicine to the mobile phone 5 based on the transmitted medicine-taking information. For example, when the medicine is taken at a rate of less than 80%, when the medicine is not taken for three consecutive days, when the medicine-taking information is not transmitted for three consecutive days, or the like, the medicine-taking condition judgment part 7a transmits the warning mail. This can remind the patient of having forgot to take the medicine, having deluded himself/herself, or the like.

When an abnormality is found in the blood pressure data transmitted from the data transfer device 3 via the mobile phone 5, the abnormal blood pressure value treatment part 7b provided in the server 7 transmits an abnormality report mail to the doctor's computer 8. The doctor's computer 8 transmits a treatment instruction to the abnormal blood pressure value treatment part 7b based on the abnormality report mail. The abnormal blood pressure value treatment part 7b transmits a treatment instruction mail to the mobile phone 5 based on the treatment instruction transmitted from the doctor's computer 8.

In the server 7, a plurality of kinds of treatment instruction mails are prepared in advance. The plurality of kinds of treatment instruction mails are graded in accordance with the degree of the abnormality of the blood pressure data. For example, in the case of testing an antihypertensive medicine whose dosage is to be increased/decreased every four weeks in principle, the following three mails are prepared.
1) A treatment instruction mail that instructs the patient to change (increase or decrease) a dosage for the subsequent times (from the fourth week).
2) A treatment instruction mail that instructs the patient to change (increase or decrease) a dosage ahead of time.
3) A treatment instruction mail that instructs the patient to visit the hospital immediately.

The abnormal blood pressure value treatment part 7b selects one of the plurality of kinds of treatment instruction mails prepared in advance based on the treatment instruction transmitted from the doctor's computer 8, and transmits the selected mail to the mobile phone 5. In this manner, the doctor is informed of the abnormal value automatically, which helps his/her patient to feel secure.

Further, the patient can transmit the harmful symptom information indicating his/her own physical harmful symptom to the server 7 using the mobile phone 5. Examples of the harmful symptom information include information on headaches, dizziness, nausea, hives, and the like.

The harmful symptom treatment part 7c provided in the server 7 transmits a harmful symptom report to the doctor's computer 8 based on the harmful symptom information transmitted from the mobile phone 5. The doctor's computer 8 transmits a treatment instruction to the harmful symptom treatment part 7c by return based on the harmful symptom report transmitted from the harmful symptom treatment part 7c. The harmful symptom treatment part 7c transmits a treatment instruction mail to the mobile phone 5 based on the treatment instruction from the doctor's computer 8.

In this data collection system, since the data transfer device 3 is operated by the power source 21 constituted by a dry battery, it can be handled conveniently. The mobile phone to be connected to the data transfer device 3 is in widespread use and is easily available, and can be handled conveniently due to its small size. Therefore, for example, it is easy for a patient in a remote area to measure his/her own blood pressure with the sphygmomanometer 1 three or four times each day and to transmit the sphygmomanometer data to the server 7 connected to the Internet once each day on that night. Alternatively, it also is easy to transmit one day's data to the server 7 at one time.

The operating procedure for the transmission is as follows, for example. That is, initially the mobile phone 5 is turned on, and then the data transfer device 3 and the sphygmomanometer 1 are turned on. After that, the data transfer device 3 and the mobile phone 5 are connected to each other via the transfer cable 4, and the data transfer device 3 and the sphygmomanometer 1 are connected to each other via the serial cable 2. Then, when the push button switch 16 of the data transfer device 3 is pushed, the blood pressure data measured on that day are transmitted automatically from the sphygmomanometer 1 to the server 7 via the mobile phone 5 and the Internet 6.

The sphygmomanometer data and the serial number transferred from the mobile phone 5 to the server 7 over the Internet 6 are constituted by numbers. Thus, even if these data are intercepted on the Internet 6, the ID of the patient is not revealed. In this manner, the patient's privacy can be protected.

The doctor can identify the patient only after checking the serial number and the patient ID transferred from the server. Since the serial number is assigned to each data transfer device 3, no means is required to identify the sphygmomanometer and the mobile phone. For example, there is no need to input an ID to the sphygmomanometer and the mobile phone, resulting in a simple operation. Further, since one data transfer device can correspond to one patient, the sphygmomanometer and the mobile phone can be used by a plurality of patients. Further, the doctor can analyze the blood pressure data collected in the server 7 instantly with the doctor's computer 8, and therefore can be aware of any abnormal values in the blood pressure data immediately. As a result, treatment and the like can be provided to the patient quickly.

In the above-mentioned embodiment, the data transfer device 3 is connected to the sphygmomanometer 1. However, the present invention is not limited thereto. Any electric devices that can transfer data to be handled by means of serial communications can be connected to the data transfer device 3 of the present embodiment so as to achieve the same effect. For example, the data transfer device may be connected to other medical instruments such as a pulsimeter for measuring a pulse rate of a patient, a dialyzer, a glucometer, and the like.

Alternatively, the data transfer device 3 may be connected to an electric meter for measuring a consumption of electricity, a water meter for measuring a consumption of tap water, or the like. Alternatively, the data transfer device 3 may be connected to a production facility for producing goods so as to transfer log data concerning an operating state of the production facility Alternatively, the data transfer device 3 may be connected to a household electric appliance so as to transfer data indicating an operating state of the household electric appliance, such as data indicating whether or not the battery is exhausted.

### Industrial Applicability

The data collection system according to the present invention can collect simply device data such as blood pressure data acquired by a device such as a sphygmomanometer provided in a remote area, via the Internet.

## Claims

1. A data collection system comprising: a server connected to the Internet; a plurality of data acquisition target devices; and a plurality of data transfer devices that are capable of being connected to the data acquisition target devices, respectively, wherein device data acquired by each data transfer device from each data acquisition target device are transferred to the server via the Internet using a mobile phone,
the data transfer device including:
a data acquisition part for acquiring the device data from the data acquisition target device;
a data transfer part for combining transmission instruction data with the device data so as to generate transfer data, and transferring the transfer data to the mobile phone; and
a control part for allowing the data acquisition part to execute the operation of acquiring the device data and allowing the data transfer part to execute the operation of transferring the transfer data,
wherein the transmission instruction data correspond to an instruction to the mobile phone to perform an operation of transmitting the device data to a predetermined server via the Internet.

2. The data collection system according to claim 1, wherein the data transfer device includes a starting switch, and the control part starts the operations of the data acquisition part and the data transfer part when the starting switch is operated.

3. The data collection system according to claim 1 or 2, wherein the data transfer device further includes a memory for storing a serial number for identifying the data transfer device mutually, and the data transfer part adds the serial number to the device data so as to generate data to be transmitted to the server.

4. The data collection system according to any one of claims 1 to 3, wherein the data transfer part includes an emulator for converting data into an emulation code in accordance with a keyboard emulation format for operating the mobile phone, so as to convert the transfer data into the emulation code and to transfer the converted data.

5. The data collection system according to claim 4, wherein the data transfer part includes a data generation part and a transfer processing part, the data generation part generates data including the device data and the transmission instruction data and supplies the generated data to the transfer processing part, and the transfer processing part converts the data supplied from the data generation part into the emulation code and outputs the converted data from a data transfer terminal.

6. The data collection system according to claim 1, wherein the control part has a function of detecting whether or not a good connection state is established between the data acquisition target device and the mobile phone, and a display part is provided so that whether or not a good connection state is established is shown by a blinking state controlled by the control part.

7. The data collection system according to claim 1, wherein the data acquisition target device is a medical instrument, an electric meter for measuring a consumption of electricity, a water meter for measuring a consumption of tap water, a production facility that is capable of outputting log data, or a household electric appliance that is capable of outputting data indicating an operating state.

8. The data collection system according to claim 7, wherein the medical instrument is a sphygmomanometer, a pulsimeter, or a glucometer.

9. The data collection system according to claim 7,
wherein the data acquisition target device is a medical instrument,
medicine-taking information indicating whether or not the patient takes a predetermined medicine is capable of being transmitted to the server using the mobile phone, and
the server includes a medicine-taking condition judgment part for accumulating the medicine-taking information and transmitting a warning mail concerning the taking of the medicine to the mobile phone based on the medicine-taking information.

10. The data collection system according to claim 9, wherein the medicine-taking condition judgment part transmits the warning mail when the medicine is taken at a rate less than a predetermined rate, or when the medicine is not taken for a predetermined number of consecutive days.

11. The data collection system according to claim 1, further comprising a doctor's computer connected to the Internet for analyzing the data accumulated in the server,
wherein the data acquisition target device includes a sphygmomanometer for measuring patient's blood pressure, and the data include blood pressure data measured by the sphygmomanometer,
the server includes an abnormal blood pressure value treatment part for transmitting an abnormality report mail to the doctor's computer when an abnormality is found in the blood pressure data,
the doctor's computer transmits a treatment instruction to the abnormal blood pressure value treatment part based on the abnormality report mail, and
the abnormal blood pressure value treatment part transmits a treatment instruction mail to the mobile phone based on the treatment instruction.

12. The data collection system according to claim 11, wherein the abnormal blood pressure value treatment part transmits one of a plurality of kinds of treatment instruction mails prepared in advance to the mobile phone.

13. The data collection system according to claim 11, wherein the treatment instruction mail includes a description of instructing the patient to change a dosage of a medicine to take.

14. The data collection system according to claim 1, further comprising a doctor's computer connected to the Internet for analyzing the data accumulated in the server,
wherein the data acquisition target device includes a sphygmomanometer for measuring patient's blood pressure, and the data include blood pressure data measured by the sphygmomanometer,
harmful symptom information indicating patient's harmful symptom is capable of being transmitted from the mobile phone to the server,
the server includes a harmful symptom treatment part for transmitting a harmful symptom report to the doctor's computer based on the harmful symptom information,
the doctor's computer transmits a treatment instruction to the harmful symptom treatment part based on the harmful symptom report, and
the harmful symptom treatment part transmits a treatment instruction mail to the mobile phone based on the treatment instruction.

15. A data collection method for collecting data by using a server connected to the Internet, a plurality of data acquisition target devices, a plurality of data transfer devices that are capable of being connected to the data acquisition target devices, respectively, and a mobile phone that is capable of being connected to the Internet, the method comprising transferring device data acquired from each data acquisition target device from the mobile phone to the server via the Internet,
each of the data transfer devices including:
a data acquisition part for acquiring the device data from the data acquisition target device;
a data transfer part for combining transmission instruction data with the device data so as to generate transfer data, and transferring the transfer data to the mobile phone; and
a control part for allowing the data acquisition part to execute the operation of acquiring the device data and allowing the data transfer part to execute the operation of transferring the transfer data,
wherein the transmission instruction data correspond to an instruction to the mobile phone to perform an operation of transmitting the device data to a predetermined server via the Internet.
